# FASCICULE DE BREVET EUROPEEN

(11) **EP 2 903 495 B1**
(45) Date de publication et mention de la délivrance du brevet: **12.12.2018**
(21) Numéro de dépôt: 13771538.9
(22) Date de dépôt: 04.10.2013
(51) Int. Cl.: A61B 1/012, A61B 1/00, A61B 1/05, A61B 1/313

(54) **SYSTÈME D'IMAGERIE MULTI-VISION POUR CHIRURGIE LAPAROSCOPIQUE**
MEHRFACHANSICHTSBILDGEBUNGSSYSTEM FÜR LAPAROSKOPISCHE CHIRURGIE
MULTI-VIEW IMAGING SYSTEM FOR LAPAROSCOPIC SURGERY

(30) Priorité: 05.10.2012 FR 1259489
(43) Date de publication de la demande: 12.08.2015
(73) Titulaire: Centre National de la Recherche Scientifique (CNRS), 75016 Paris (FR); Université Joseph Fourier - Grenoble 1, 38400 Saint-Martin-d'Hères (FR); Centre Hospitalier Universitaire Grenoble, 38700 La Tronche (FR)
(72) Inventeur: VOROS, Sandrine, 38000 Grenoble (FR); CINQUIN, Philippe, 38330 St Nazaire Les Eymes (FR); FOUARD, Céline, 38100 Grenoble (FR); TAMADAZTE, Brahim, 25000 Besançon (FR)
(74) Mandataire: Hautier IP
(86) Numéro de dépôt international: PCT/EP2013/070740
(87) Numéro de publication internationale: WO 2014/053649

(56) Documents cités:
- WO-A1-93/25138
- CN-A- 102 058 375
- US-A1- 2009 259 097
- US-A1- 2011 306 832
- US-A1- 2012 065 468

## Description

### DOMAINE DE L'INVENTION

La présente invention concerne le domaine de l'imagerie médicale, plus particulièrement l'imagerie dans le cadre de chirurgie laparoscopique.

### ETAT DE LA TECHNIQUE

La chirurgie laparoscopique, appelée également chirurgie mini-invasive (MIS, selon l'acronyme anglais « Minimal invasive Surgery »), utilisée notamment pour effectuer des opérations intra-abdominales ou intra-thoraciques, nécessite de petites incisions, en général inférieures à 1 cm, en comparaison des incisions larges requises en laparotomie. Cette approche permet de diminuer les pertes de sang et douleurs postopératoires, d'avoir de faibles hémorragies, de réduire le temps de récupération tout en offrant une meilleure cicatrisation.

La chirurgie laparoscopique appartient au champ plus large de l'endoscopie qui utilise des systèmes d'imagerie permettant de visualiser le champ opératoire. Un endoscope peut consister en un ensemble de lentilles agencées dans un tube et connecté à une caméra vidéo, ou être un laparoscope numérique dans lequel un capteur CCD (acronyme anglais de « Charge-Coupled Device » pour « dispositif à transfert de charge ») est positionné à l'extrémité active du laparoscope destiné à acquérir les images.

Dans les techniques laparoscopiques, les chirurgiens utilisent souvent de deux à quatre instruments spécifiques introduits dans le corps du patient à travers autant de trocarts. Le chirurgien porte un instrument dans chacune de ses mains, de sorte que l'endoscope doit, en général, être tenu par un assistant. De ce fait, le chirurgien n'a pas une liberté totale pour commander la visualisation de l'endoscope.

Des robots ont donc été développés pour permettre au chirurgien de contrôler lui-même, par commande vocale par exemple, l'orientation et le zoom de l'endoscope, ceci de manière précise, suivant ses besoins pendant une intervention sans avoir à communiquer avec un assistant. On peut citer par exemple le robot porte-endoscope ViKY® commercialisé par la société Endocontrol, ou encore le robot médical daVinci® commercialisé par la société Intuitive Surgical. Même si ces robots ont permis d'améliorer fortement la pratique des chirurgiens, il reste des points à améliorer, notamment en ce qui concerne la limitation du champ de vision offert par ces dispositifs, sa qualité, ainsi que l'accès aux zones cachées dans le champ opératoire.

Parmi les développements de systèmes de visualisation pour la chirurgie laparoscopique, il a été proposé des dispositifs de vision stéréoscopique. Par exemple, le document US 5,305,121 propose de remplacer le traditionnel mono-endoscope par un stéréo-endoscope comprenant un tube à l'intérieur duquel peut coulisser un éclairage à base de fibres optiques et un agencement de deux caméras CCD montées sur l'éclairage. Au départ, les caméras sont prépositionnées dans le tube qui contient également la source de lumière. Une fois ce tube introduit à l'intérieur de la cavité abdominale à travers un trocart, le praticien pousse les caméras à l'extérieur du tube par l'intermédiaire de l'éclairage. Ensuite, les caméras peuvent être orientées l'une par rapport à l'autre à l'aide d'actionneurs AMF (acronyme de « Alliages à Mémoire de Forme », ou SMA selon l'acronyme anglais « Shape-Memory Alloy »). Un tel dispositif est destiné à fournir au praticien des informations plus complètes sur le champ opératoire grâce à une vision stéréoscopique. Ce dispositif de vision stéréoscopique comprend toutefois les mêmes limites qu'un mono-endoscope, notamment en termes de résolution, profondeur de champ, et champ de vision. En outre, l'agencement proposé impose un déploiement de caméras dans le champ opératoire qui n'est pas contrôlé et qui peut venir heurter certains organes internes et venir compromettre l'intervention chirurgicale.

Le document US 6,614,595 propose un système d'imagerie qui permet d'élargir le champ de vision habituel, puisque ce document propose un endoscope combinant deux optiques agencées pour offrir une vision stéréoscopique à une troisième optique permettant une vision plus large. Plus précisément, il est proposé d'intégrer dans un même corps un ensemble de lentilles en série, permettant d'amener l'image de la partie distale de l'endoscope vers la partie externe sur laquelle est fixée une caméra. L'architecture monobloc proposée, où le système de vision global est totalement dépendant du système de vision stéréoscopique, en termes d'orientation et de déplacement suivant l'axe optique de l'endoscope (zoom avant et zoom arrière) est présentée comme permettant de garantir un champ de vision globale large tout en ayant une qualité d'image suffisante pour une visualisation précise pour les caméras déportées permettant la vision stéréoscopique. L'architecture proposée est toutefois très complexe, et très contrainte par la taille du corps utilisé. Un tel système n'est par ailleurs pas optimal pour garantir au praticien une vision globale lorsqu'il désire effectuer un agrandissement avec la vision stéréoscopique.

Le document US 2012/0065468 propose également un système d'imagerie de type endoscope, adapté pour la colonoscopie notamment, qui offre un champ de vision élargi par rapport aux endoscopes traditionnels. En effet, il est prévu un endoscope ayant un corps cylindrique avec à son extrémité distale, de façon classique, un premier élément de vision, tel qu'une caméra, l'endoscope comprenant en outre un ou plusieurs éléments secondaires de vision fixés sur les parois latérales du corps cylindrique, pour offrir une vision latérale en plus de la vision centrale. Ces éléments de vision latéraux donnent des informations supplémentaires au praticien lors de l'avancé de l'endoscope dans le colon, et permettent éventuellement de visualiser des éléments spécifiques à traiter sur les parois internes dudit colon. Un tel système n'est toutefois pas prévu pour des chirurgies laparoscopiques où il ne présente pas d'intérêt puisque la vision latérale n'est pas utile pour l'opération en tant que telle. En outre, un tel dispositif ne permet pas d'offrir au praticien une vision globale du champ opératoire combinée à une vision spécifique d'un élément choisi au sein de ce champ opératoire.

Le document US 2011/0306832 propose un endoscope permettant une visualisation du champ opératoire selon différents points de vue, ou une visualisation tridimensionnelle dudit champ opératoire. A cette fin, il est proposé un endoscope ayant une extrémité distale à laquelle sont fixées trois bras déployables, chaque bras portant un capteur vidéo pour permettre la visualisation du champ opératoire pendant l'opération. Cet endoscope peut en outre éventuellement comprendre une caméra au niveau de son axe central, cette caméra centrale étant utilisée uniquement pour faciliter l'insertion de l'endoscope jusqu'au champ opératoire, et n'étant pas utilisée pendant l'opération en tant que telle. Un tel système de visualisation a une toutefois conception complexe et très spécifique. En outre, il ne permet pas d'offrir au praticien une vision globale du champ opératoire combinée à une vision spécifique d'un élément choisi au sein de ce champ opératoire.

Un but de la présente invention est donc de proposer un système d'imagerie pour chirurgie laparoscopique qui permet de résoudre au moins l'un des inconvénients précités.

En particulier, un but de la présente invention est de proposer un système d'imagerie pour chirurgie laparoscopique qui offre au praticien un champ de visualisation élargi tout en lui permettant des agrandissements sur une zone spécifique du champ opératoire.

Encore un but de la présente invention est de proposer un système d'imagerie pour chirurgie laparoscopique multi-vision, simple à utiliser et offrant une sécurité accrue vis-à-vis du patient.

Un autre but de la présente invention est de proposer un système d'imagerie pour chirurgie laparoscopique multi-vision offrant une vision globale de la cavité abdominale. Ceci permet de visualiser d'une manière plus large, par exemple, l'introduction des instruments dans la cavité abdominale.

Encore un autre but de la présente invention est de proposer un système d'imagerie pour chirurgie laparoscopique, qui peut être adapté aux endoscopes standards existants.

### EXPOSE DE L'INVENTION

A cette fin, on propose un système d'imagerie multi-vision pour la chirurgie laparoscopique, caractérisé en ce qu'il comprend :
- Un organe tubulaire, ledit organe tubulaire ayant un axe longitudinal, une extrémité distale et une extrémité proximale ;
- Un premier dispositif d'imagerie ayant un corps longitudinal et une extrémité active pour l'acquisition d'images, le premier dispositif d'imagerie étant destiné à être inséré à travers l'organe tubulaire avec l'extrémité active en saillie par rapport à l'extrémité distale, et le premier dispositif d'imagerie étant mobile dans l'organe tubulaire suivant une translation selon l'axe longitudinal et/ou suivant une rotation autour de l'axe longitudinal ;
- Un deuxième dispositif d'imagerie comprenant au moins deux caméras montées chacune sur un organe de support et étant déplaçables par rapport à l'organe tubulaire entre :
   ∘ une position escamotée dans laquelle les caméras sont positionnées à l'intérieur de l'organe tubulaire, et
   ∘ une position déployée dans laquelle les caméras sont positionnées à l'extérieur de l'organe tubulaire au niveau de l'extrémité distale, les caméras étant agencées de part et d'autre de l'axe longitudinal de l'organe tubulaire et étant maintenues fixes par rapport à l'organe tubulaire par des moyens de maintien, de manière à suivre tout mouvement de l'organe tubulaire.

Des aspects préférés mais non limitatifs de ce système d'imagerie, pris seuls ou en combinaison, sont les suivants :
- les moyens de maintien sont au moins en partie formés par la paroi des organes de support destinée à venir en appui sur le corps longitudinal du premier dispositif d'imagerie, en position déployée.
- les moyens de maintien comprennent des organes de rappel pour maintenir les caméras en appui contre l'organe tubulaire, en position déployée.
- en position escamotée, les caméras sont alignées à l'intérieur de l'organe tubulaire selon l'axe longitudinal.
- en position escamotée, les caméras sont positionnées à l'intérieur de l'organe tubulaire en regard l'une de l'autre de part et d'autre de l'axe longitudinal.
- en position déployée, les caméras sont positionnées de sorte à avoir un axe optique parallèle à l'axe longitudinal.
- en position déployée, les caméras sont positionnées de sorte que les axes optiques des caméras forment un angle compris entre 6° et 15°.
- en position déployée, les caméras sont positionnées de sorte que les axes optiques des caméras sont séparés d'une distance comprise entre 10 millimètres et 30 millimètres.
- en position déployée, les caméras sont positionnées de sorte que les axes optiques des caméras et l'axe longitudinal sont dans un même plan.
- les organes de support sont montés en rotation par rapport à l'organe tubulaire, les organes de support comprenant un ergot d'actionnement permettant au corps longitudinal de déplacer en rotation les organes de support en vue de leur déploiement lors de l'insertion du premier dispositif d'imagerie dans l'organe tubulaire.
- les organes de support sont montés en translation par rapport à l'organe tubulaire, les organes de support comprenant un ergot d'actionnement permettant au corps longitudinal de déplacer en translation les organes de support en vue de leur déploiement lors de l'insertion du premier dispositif d'imagerie dans l'organe tubulaire.
- l'organe tubulaire comprend deux rails et les organes de support des caméras comprennent un ergot de guidage destiné à coopérer avec les rails pour une translation guidée des organes de support des caméras par rapport à l'organe tubulaire.
- l'organe tubulaire est un trocart destiné à être positionné à travers une incision pratiquée dans le corps d'un patient.
- l'organe tubulaire est un adaptateur interne destiné à être inséré à l'intérieur d'une portion tubulaire d'un trocart, le diamètre interne de l'organe tubulaire étant sensiblement le même que le diamètre externe de la portion tubulaire du trocart.
- l'organe tubulaire est un adaptateur externe à l'intérieur duquel une portion tubulaire d'un trocart est destinée à être insérée, le diamètre externe de l'organe tubulaire étant sensiblement le même que le diamètre interne de la portion tubulaire du trocart.
- le premier dispositif d'imagerie est un endoscope.

### DESCRIPTION DES FIGURES

D'autres caractéristiques et avantages de l'invention ressortiront encore de la description qui suit, laquelle est purement illustrative et non limitative et doit être lue en regard des dessins annexés, sur lesquels :
- la figure 1 est une représentation d'un champ opératoire avec des outils chirurgicaux et le système d'imagerie multi-vision selon l'invention ;
- la figure 2 est une représentation en perspective du système d'imagerie multi-vision selon l'invention ;
- les figures 3A, 3B et 3C représentent la structure et le fonctionnement du système d'imagerie multi-vision selon un premier mode de réalisation de l'invention ;
- les figures 4A, 4B et 4C représentent la structure et le fonctionnement du système d'imagerie multi-vision selon un deuxième mode de réalisation de l'invention ;
- les figures 5 et 6 représentent la structure du système d'imagerie multi-vision selon un troisième mode de réalisation de l'invention ;
- les figures 7 et 8 représentent la structure du système d'imagerie multi-vision selon un quatrième mode de réalisation de l'invention ;
- la figure 9 représente une séquence d'images prises par un endoscope traditionnel lors d'une reproduction d'une tâche de suture en laparoscopie ;
- la figure 10 représente une séquence d'images prises par le système d'imagerie multi-vision selon l'invention lors d'une reproduction d'une tâche de suture en laparoscopie.

### DESCRIPTION DETAILLEE DE L'INVENTION

Le système d'imagerie proposé consiste à combiner un premier dispositif d'imagerie classiquement utilisé par les chirurgiens lors d'interventions laparoscopiques, par exemple l'endoscopie, avec un deuxième dispositif d'imagerie offrant une vision globale du champ opératoire dans lequel le chirurgien intervient. Ce deuxième dispositif d'imagerie a pour objet de fournir au chirurgien des informations supplémentaires concernant l'environnement opératoire, pour faciliter son intervention sans avoir à nécessairement intervenir sur le premier dispositif d'imagerie, notamment pour le déplacer. Le chirurgien s'affranchit par exemple des traditionnels déplacements (introduction et extraction) de l'endoscope afin de faire un zoom avant ou arrière par rapport au champ opératoire. Le nouveau système d'imagerie garantit une vision globale du site opératoire tandis que l'endoscope assure une vision plus locale et précise de ce site.

Dans le reste de la description, le premier dispositif d'imagerie décrit est un endoscope 20 que le chirurgien peut insérer dans le champ opératoire à travers un trocart 10 positionné à travers une incision pratiquée chez le patient. Toutefois, tout dispositif d'imagerie ayant des caractéristiques similaires aux endoscopes, notamment en termes de forme, pourrait être utilisé. En particulier, le premier dispositif d'imagerie pourrait fonctionner sur des technologies d'imagerie infrarouge ou à fluorescence ou échographique.

L'endoscope 20 a un corps longitudinal 21 et une extrémité active 22 pour l'acquisition d'images. Cette extrémité active 22 correspond à la partie de l'endoscope 20 destinée à être dans le champ opératoire pour l'observation. Les endoscopes traditionnels utilisent un ensemble de lentilles optiques positionnées dans le corps longitudinal 21 depuis l'extrémité active 21 jusqu'à l'extrémité opposée où est généralement positionnée une caméra 40, connectée à un système d'affichage, tel qu'un écran, pour visualisation par le chirurgien. Avec la miniaturisation de l'électronique, il existe aujourd'hui des endoscopes 20, dits endoscopes électroniques, pour lesquels le capteur de la caméra est directement positionné au niveau de l'extrémité active ce qui permet d'éviter d'avoir un système à lentilles complexe.

Lors d'une intervention, l'endoscope 20 est inséré à travers un trocart 10 qui assure l'étanchéité entre l'incision effectuée sur le patient et l'endoscope. Le trocart assure également le rôle d'intermédiaire pour l'introduction du gaz CO2 dans la cavité abdominale afin de créer un espace de travail pour le chirurgien. En outre, le trocart 10 permet de guider les mouvements de l'endoscope 20 par rapport au patient. Il peut être considéré que le trocart est une liaison de type rotule par rapport au corps du patient au niveau de l'élément d'étanchéité 11 positionné à l'incision pratiquée dans le corps du patient. L'endoscope 20 a des dimensions adaptées pour que le corps longitudinal 21 puisse être inséré à travers le trocart 10, en étant mobile dans le trocart 10 suivant une translation selon l'axe longitudinal L et/ou suivant une rotation autour de l'axe longitudinal L. Ainsi, la translation de l'endoscope 20 par rapport au trocart 10 permet au praticien de faire un zoom avant (correspondant à un agrandissement), puisqu'il rapproche l'extrémité active de la zone à observer. La rotation autour de l'axe longitudinal L permet une rotation propre de l'endoscope 20 pour changer l'orientation de l'image. Enfin, l'endoscope 20 peut être positionné selon l'importe quel angle d'observation par l'intermédiaire de la liaison rotule formée au niveau de l'incision (point d'insertion du trocart 10).

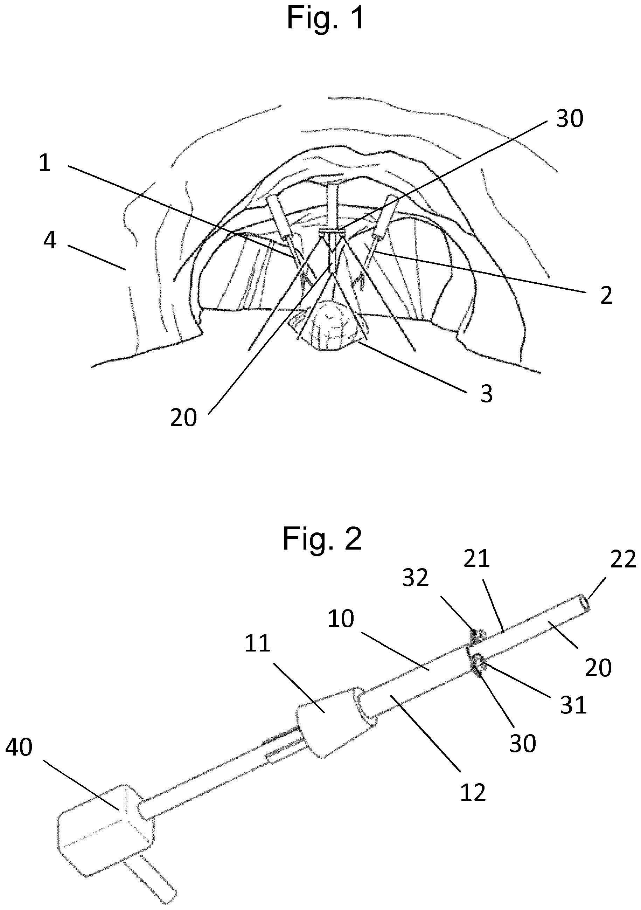

Le système d'imagerie proposé présente la particularité d'avoir un deuxième dispositif d'imagerie 30 qui est prévu pour offrir au chirurgien une autre vision du champ opératoire que celle offerte par l'endoscope 20. Plus particulièrement, le deuxième dispositif d'imagerie 30 est prévu pour offrir une vision globale du champ opératoire tandis que le premier dispositif d'imagerie 20 offre une vision plus localisée, en fonction des besoins du chirurgien (l'endoscope étant libre de mouvements le long et autour de son axe optique par rapport au deuxième dispositif d'imagerie), d'une zone du champ opératoire. Avec un dispositif d'affichage des images adapté, le praticien peut donc à la fois avoir un aperçu général du champ opératoire tout en restant zoomé sur la zone d'intervention par exemple. Cela est très utile par exemple lorsque le chirurgien doit introduire des outils (1,2) dans le champ opératoire 3 ou les retirer du champ opératoire 3, puisqu'il n'a pas besoin de déplacer l'endoscope pour voir le déplacement de ces outils chirurgicaux dans le champ opératoire (voir figure 1). En outre, des déplacements intempestifs de l'endoscope risquent de salir l'optique par exemple, ce qui nécessiterait de sortir régulièrement l'endoscope de la cavité abdominale 4, le nettoyer, puis le réinsérer dans le patient, entraînant des temps opératoires supplémentaires. Cela permet également au chirurgien de pouvoir très précisément estimer la position relative des outils chirurgicaux (1,2) utilisés, sans modifier la position ou l'orientation du système d'imagerie.

Le deuxième dispositif d'imagerie 30 comprend de préférence au moins deux caméras (31;32) qui sont déplaçables par rapport au trocart 10 ou un autre organe tubulaire, formant adaptateur, destiné à être couplé au trocart 10 au niveau de la portion tubulaire présente dans le champ opératoire. Plus précisément, les caméras (31;32) sont déplaçables entre une position escamotée prévue pour l'insertion et/ou le retrait du système d'imagerie dans le champ opératoire, et une position déployée pendant l'intervention permettant d'avoir les différentes visions mentionnées plus haut. De préférence encore, les deux caméras (31;32) sont montées mobiles sur le trocart 10 ou l'organe tubulaire, liées de manière rigide. Ces caméras (31;32) restent toutefois libres par rapport aux déplacements de l'endoscope suivant son axe optique (zoom avant et zoom arrière). Plus précisément, les caméras (31;32) suivent les déplacements en rotation de l'endoscope traditionnel (rotation par rapport au point d'insertion) mais elles restent fixes lorsqu'il s'agit de déplacer l'endoscope pour faire un zoom avant ou arrière. Ainsi, l'association des deux dispositifs d'imagerie permet à la fois de garantir une vision localisée avec l'endoscope lorsqu'il est enfoncé et une vision plus généralisée (dans tous les cas) grâce au deuxième dispositif d'imagerie 30. Dans la position escamotée, les caméras sont prévues pour être positionnées à l'intérieur d'un organe tubulaire 12, à savoir le trocart ou l'organe tubulaire formant adaptateur. Ainsi, lorsque cet organe tubulaire 12 est inséré dans le champ opératoire, il ne risque pas ou peu de venir en contact de manière non souhaitée avec des organes présents dans le champ opératoire. De la même façon, en position escamotée, le retrait de l'organe tubulaire 12 du champ opératoire n'est pas gêné par les caméras (31;32).

Dans la position déployée, les caméras (31;32) sont positionnées à l'extérieur de l'organe tubulaire 12 au niveau de l'extrémité distale de cet organe tubulaire 12, c'est-à-dire l'extrémité la plus proche du champ opératoire. Les caméras (31;32) sont en outre agencées de part et d'autre de l'axe longitudinal L de l'organe tubulaire 12. De préférence, la caméra 31 est positionnée en symétrique de l'autre caméra 32 par rapport à l'axe longitudinal L.

Par ailleurs, en position déployée, les caméras (31;32) sont maintenues fixes par rapport à l'organe tubulaire 12 de manière à être solidaires de l'organe tubulaire 12, afin notamment d'avoir des déplacements identiques à ceux de l'organe tubulaire 12. Des moyens de maintien adaptés permettent de rendre les caméras (31;32) solidaires de l'organe tubulaire 12 en position déployée, tout en autorisant un déplacement des caméras (31;32) depuis ou vers la position escamotée. Comme on le verra plus loin, ces moyens de maintien peuvent notamment être formés en partie par les organes de support 33 sur lesquels sont montées les caméras (31;32), en coopération par exemple avec l'endoscope 20.

Le couplage des caméras (31;32) à l'organe tubulaire 12 - pouvant par exemple correspondre au trocart 10 - permet d'avoir un champ de vision global qui est orienté selon l'orientation du trocart autour de la liaison rotule, qui correspond également à l'orientation de l'endoscope 20 en translation dans l'organe tubulaire 12. Toutefois, puisque les caméras (31;32) ne sont pas couplées à l'endoscope 20, cela permet de rendre indépendants certains déplacements de l'endoscope 20 par rapport au deuxième dispositif d'imagerie 30, à savoir la translation selon l'axe longitudinal L de l'organe tubulaire 12 et la rotation autour de ce même axe longitudinal L. De manière très avantageuse, l'endoscope 20 peut donc être rapproché de la zone d'intervention, pour faire un zoom sur un organe particulier par exemple, tout en conservant une vision globale du champ opératoire inchangée.

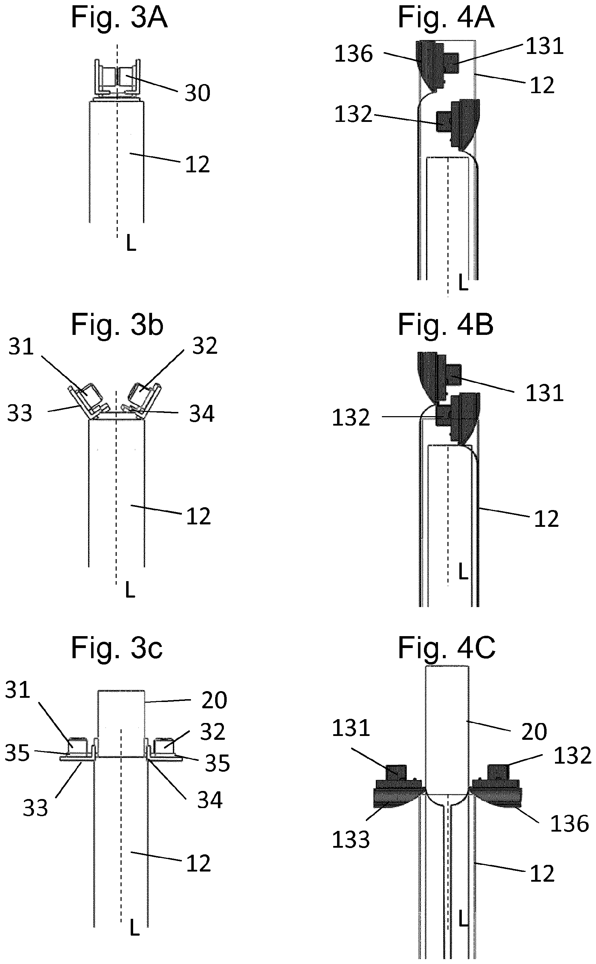

Comme illustré à la figure 2, les caméras (31;32) du deuxième dispositif d'imagerie sont de préférence positionnées, en position déployée, de part et d'autre de l'organe tubulaire 12, et donc de l'endoscope 20. Les caméras (31;32) ont dans ce cas un agencement « en lunettes » autour de l'endoscope 20. On retrouve cet agencement « en lunettes » illustré aux figures 3C et 4C.

Le fait d'utiliser au moins deux caméras (31;32) de part et d'autre de l'organe tubulaire 12 présente de nombreux avantages. Comme illustré à la figure 1, cette disposition spécifique des caméras (31;32) permet par exemple d'apercevoir le bout de l'endoscope 20, les trocarts latéraux (trocarts pour les instruments) ainsi que les instruments dès leur introduction dans l'abdomen du patient. Ceci permet d'éviter les mauvaises surprises, souvent rencontrées par le praticien à l'introduction des différents outils nécessaires pour la chirurgie laparoscopique, et réduit considérablement le risque d'accidents, par exemple une perforation accidentelle d'un organe sain.

Par ailleurs, l'utilisation des caméras agencées « en lunettes » est très naturelle pour le chirurgien, puisque aucun recalage entre les différentes images n'est nécessaire, ce qui accélère l'apprentissage du praticien à l'utilisation de ce système. On peut en effet envisager un dispositif d'affichage avec un premier écran affichant l'image d'une première caméra 31, un deuxième écran - à côté du premier écran - affichant l'image de l'endoscope 20, et un troisième écran - à côté du deuxième écran - affichant l'image de la deuxième caméra 32. Selon un autre mode de réalisation, le système d'affichage peut être couplé à une unité de traitement d'images prévue pour recaler et fusionner les images des caméras (31;32) (méthode appelée mosaicing dans le domaine de la vision par ordinateur) afin de créer un champ de vision élargi autour de l'image de l'endoscope 20.

En position déployée, les caméras (31;32) sont donc de préférence fixées par rapport à l'organe tubulaire 12 de sorte que leurs axes optiques soient parallèles à l'axe longitudinal L de l'organe tubulaire 12, correspondant également à l'axe optique de l'endoscope 20. Par ailleurs, les axes optiques des caméras (31;32) et l'axe longitudinal L de l'organe tubulaire 12 sont de préférence dans un même plan.

Il peut également être envisagé d'utiliser les caméras (31;32) en vision stéréoscopique, auquel cas les caméras (31;32) sont agencées pour que leurs axes optiques forment, en position déployée, un angle compris entre 6° et 15°. En complément, ou de manière alternative, l'angle formé par les axes optiques des caméras, peut être recalculé après déploiement et fixation par des méthodes mathématiques de calibrage (étalonnage), afin de permettre la vision stéréoscopique recherchée.

Une configuration spéciale de ces caméras (31;32), en stéréovision, permettrait également d'utiliser des approches de reconstruction 3D pour offrir au chirurgien un environnement de navigation en 3D s'il le souhaite, tout en conservant une image endoscopique précise. Le référentiel donné par l'endoscope 20 permet en outre de faciliter la reconstruction 3D d'images.

Le deuxième dispositif d'imagerie 30 est couplé à l'organe tubulaire 12 avec des moyens permettant un déploiement, une fixation et un escamotage en vue du retrait du système d'imagerie, à la fois simple et rapide.

Avantageusement, c'est l'insertion de l'endoscope 20 dans l'organe tubulaire 12, et plus particulièrement lorsque l'extrémité active 22 de l'endoscope 20 passe l'extrémité distale de l'organe tubulaire 12, en direction du champ opératoire, qui actionne le déploiement des caméras (31;32) vers l'extérieur de l'organe tubulaire 12.

Préférentiellement, les organes de support 33 des caméras (31;32) sont montés en rotation par rapport à l'organe tubulaire 12. En outre, les organes de support 33 comprennent dans ce cas un ergot d'actionnement 34 permettant au corps longitudinal 21 de l'endoscope 20 de déplacer en rotation les organes de support 33 en vue de leur déploiement lors de l'insertion de l'endoscope 20 dans l'organe tubulaire 12.

Selon un mode de réalisation alternatif ou complémentaire, les organes de support 33 sont montés en translation par rapport à l'organe tubulaire 12. Dans ce cas, les organes de support 33 comprennent un ergot d'actionnement 34 permettant au corps longitudinal 21 de déplacer en translation les organes de support 33 en vue de leur déploiement lors de l'insertion de l'endoscope 20 dans l'organe tubulaire 12.

Ainsi, pour passer de la position escamotée à la position déployée, les organes de support 33 ont un mouvement de rotation permettant aux caméras (31;32) d'être déployées de l'intérieur de l'organe tubulaire 12, vers l'extérieur. Ce mouvement de rotation peut par ailleurs être couplé à un mouvement de translation permettant de dégager les caméras (31;32) de l'intérieur de l'organe tubulaire 12 avant leur rotation finalisant leur déploiement.

Comme indiqué plus haut, les moyens de maintien permettant - en position déployée - de maintenir les caméras (31;32) fixes par rapport à l'organe tubulaire 12, sont au moins en partie formés par la paroi des organes de support 33 destinée à venir en appui sur le corps longitudinal 21 de l'endoscope 20.

Cette coopération entre le corps longitudinal 21 de l'endoscope 20 et la paroi d'appui des organes de support 30 permet en outre de maintenir les caméras (31;32) proches de l'axe longitudinal L de l'organe tubulaire 12. De préférence, en position déployée, les caméras (31;32) sont ainsi positionnées de sorte que leurs axes optiques soient séparés d'une distance comprise entre 10 millimètres et 30 millimètres. De manière encore préférée, l'axe optique de chaque caméra est à une distance par rapport à la paroi de l'endoscope inférieure à 10 millimètres, par exemple comprise entre 2 millimètres et 8 millimètres. Dans le cas où les axes optiques des caméras forment un angle entre eux (notamment en configuration stéréoscopique), les distances précisées ci-dessus sont mesurées au niveau des centres optiques des caméras, ces centres optiques étant en général situés au même niveau que les caméras en tant que telles par rapport à l'axe longitudinal L.

Par ailleurs, les moyens de maintien comprennent de préférence des organes de rappel pour maintenir les caméras en appui contre l'organe tubulaire, en position déployée. Ces organes de rappel peuvent être des câbles mis en tension par le chirurgien une fois que les caméras sont positionnées à l'extérieur de l'organe tubulaire 12. Ces organes de rappel peuvent également se présenter sous la forme de ressorts de rappel par exemple. Selon une variante, les organes de rappel sont directement formés par les flexibles d'alimentation des caméras (31;32) ce qui simplifie l'agencement du système d'imagerie.

Les figures 3A, 3B et 3C illustrent un premier mode de réalisation du système d'imagerie proposé. Selon ce mode de réalisation, en position escamotée, les caméras (31;32) sont en regard l'une de l'autre de part et d'autre de l'axe longitudinal L. La figure 3A illustre cet agencement des caméras (31;32) lorsqu'elles viennent d'être dégagées de l'organe tubulaire 12 en vue de leur déploiement. La figure 3B illustre quant à elle la rotation des caméras (31;32) vers la position déployée illustrée à la figure 3C où les caméras (31;32) ont un agencement « en lunettes ».

Comme on le voit bien illustré sur les figures, c'est la translation de l'endoscope 20 vers l'extérieur de l'organe tubulaire 12 qui permet le déploiement des caméras (31;32) par actionnement d'une portion 34 des organes de support 33 des caméras (31;32).

Cette même portion 34 constitue également la surface d'appui de l'organe support 33 sur l'endoscope 20 qui permet de maintenir les caméras (31;32) dans une position fixe par rapport à l'organe tubulaire 12 lorsque l'endoscope 20, et donc les caméras (31;32), sont déployés.

De préférence, un circuit intégré 35 est également prévu au niveau de chaque organe de support 33 permettant de transmettre les images acquises au niveau des capteurs des caméras (31;32) vers l'unité de traitement pour affichage.

Une telle configuration où les caméras (31;32) sont en regard l'une de l'autre, de préférence à l'intérieur de l'organe tubulaire 12, en position escamotée, est possible si l'organe tubulaire 12 a un diamètre suffisamment large, ou si les caméras (31;32) sont suffisamment petites.

Dans le cas où l'espace interne de l'organe tubulaire 12 est trop petit par rapport à la taille des caméras, il peut être alors prévu un agencement selon un deuxième mode de réalisation où, en position escamotée, les caméras sont alignées à l'intérieur de l'organe tubulaire 12 selon l'axe longitudinal L. Un tel agencement est illustré aux figures 4A, 4B, 4C, qui illustrent respectivement la position escamotée, une position intermédiaire entre position escamotée et position déployée, et la position déployée.

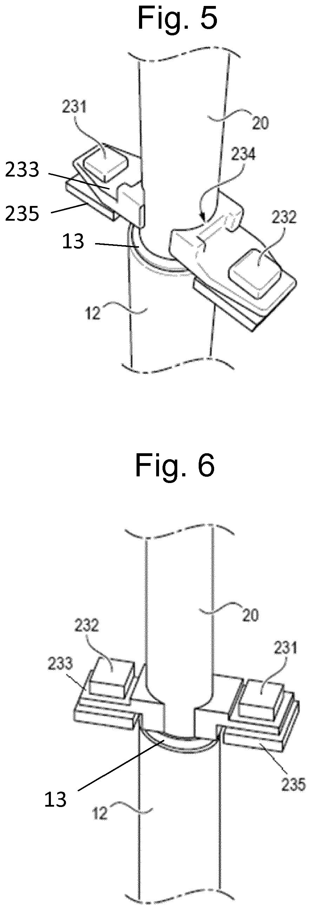

Selon encore un autre mode de réalisation illustré aux figures 5 et 6, les caméras (231;232) sont positionnées sur des organes de support 233 libres par rapport à l'organe tubulaire 12. Dans ce cas, les organes de supports 233 ont une forme très spécifique leur permettant d'être plaqués contre l'endoscope 20 et contre l'extrémité distale de l'organe tubulaire 12, de sorte que les caméras (231;232) conservent une position fixe par rapport à l'organe tubulaire 12 lorsqu'elles sont déployées. A cet égard, il peut en outre être prévu des câbles de rappel (non représentés) qui, lorsqu'ils sont mis en tension, permettent de venir contraindre les caméras (231;232) contre l'organe tubulaire 12.

De préférence, un circuit intégré 235 est également prévu au niveau de chaque organe de support 233 permettant de transmettre les images acquises au niveau des capteurs des caméras (231;232) vers l'unité de traitement pour affichage.

Selon ce mode de réalisation, en position escamotée, les caméras (231;232) et organes de support 233 associés sont prépositionnés à l'intérieur du trocart 10, au niveau de l'extrémité distale. Un organe tubulaire intermédiaire 13 est en outre positionné à l'intérieur du trocart 10, cet organe tubulaire 13 servant à limiter le jeu et renforcer l'étanchéité entre le trocart 10 et l'endoscope 20. Les câbles de mise en contrainte courent quant à eux dans l'organe tubulaire intermédiaire 13 jusqu'à l'extérieur du patient, de sorte à pouvoir être mis en tension depuis l'extérieur, au niveau proximal. Plus précisément, ces câbles sont positionnés entre le trocart 10 et la paroi externe de l'organe tubulaire intermédiaire 13. Il pourrait également être envisagé de ne pas avoir cet organe tubulaire intermédiaire 13.

Une fois que l'organe tubulaire 12 est en position dans le champ opératoire, les dispositifs d'imagerie peuvent être déployés. Pour ce faire, le praticien pousse l'endoscope 20 en direction du champ opératoire de sorte que l'extrémité active de l'endoscope soit effectivement dans le champ opératoire. Cette translation vient faire sortir les caméras (231;232) du trocart 10. Pour les mettre en position déployée, il convient ensuite de tirer sur les câbles de contrainte, ce qui rapproche les caméras (231;232) du trocart 10. La forme des organes de support 233 combinée à la contrainte des câbles vient naturellement positionner les caméras (231;232) en appui à la fois sur l'extrémité distale du trocart 12 et sur le corps longitudinal de l'endoscope 20.

Selon un autre mode de réalisation de l'invention, les caméras sont prépositionnées dans l'organe tubulaire, et sont montées en translation sur cet organe tubulaire, par l'intermédiaire de glissières. A cette fin, l'organe tubulaire peut comprendre des rails, se présentant sous la forme de fentes par exemple, et les organes de support des caméras comprennent un ergot de guidage pouvant coulisser dans le rail.

Le mode de réalisation présenté aux figures 4A, 4B et 4C intègre un tel agencement avec un système de glissières. Les ergots de guidage 136 coopèrent avec des fentes ménagées dans l'organe tubulaire 12, qui peut être le trocart ou un tube intermédiaire placé dans le trocart.

Ce système à glissières présente l'avantage de faciliter le déploiement et le positionnement des caméras. En effet, les rails coopérant avec la forme du support 133 des caméras (131;132) assurant un guidage du déploiement de ces caméras (131;132) jusqu'à leur position déployée, de préférence en position symétrique par rapport à l'axe longitudinal L de l'endoscope 20.

De préférence, un circuit intégré 135 est également prévu au niveau de chaque organe de support 133 permettant de transmettre les images acquises au niveau des capteurs des caméras (131;132) vers l'unité de traitement pour affichage.

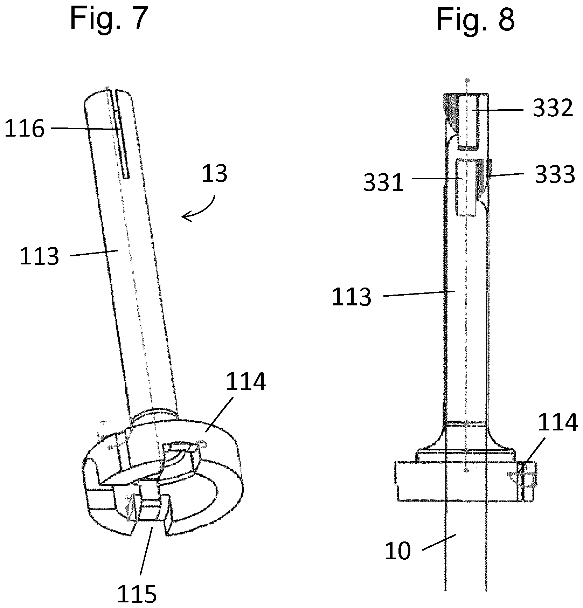

Selon encore un autre mode de réalisation tel qu'illustré aux figures 7 et 8, il est prévu un organe tubulaire 13 dans lequel la portion tubulaire du trocart 10 est destinée à être insérée. Ce mode de réalisation présente l'avantage de garantir une totale étanchéité du système global une fois positionné sur le patient.

Un trocart est généralement composé d'une portion tubulaire 12 destinée à être insérée dans le corps du patient, vers le champ opératoire, et d'un élément d'étanchéité 11 positionné au niveau de l'incision pratiquée chez le patient. Cet élément d'étanchéité 11 comprend notamment les passages pour injecter du CO2 dans le champ opératoire ou plus généralement pour assurer l'étanchéité du système.

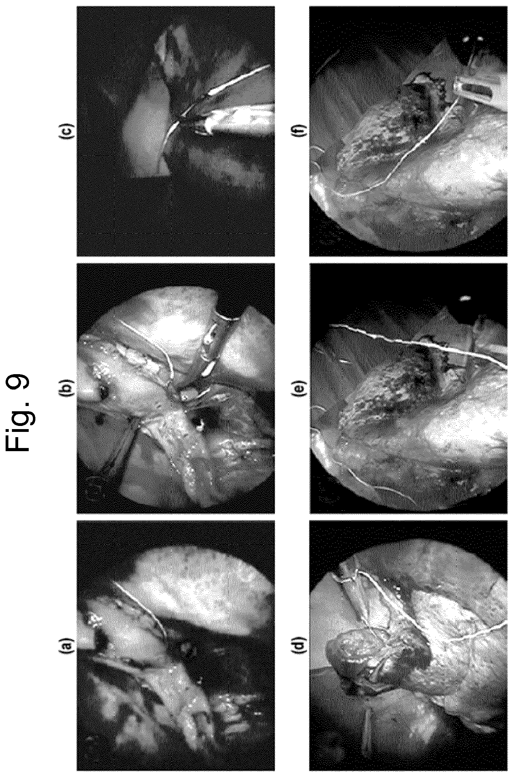

Selon le mode de réalisation présenté aux figures 8 et 9, l'organe tubulaire 13 comprend également une portion tubulaire 113 d'un diamètre légèrement supérieur au diamètre de la portion tubulaire 10 du trocart, et une bague de fixation 114 ayant une forme prévue pour recevoir l'élément d'étanchéité 11 du trocart. De préférence, la bague de fixation 114 comprend des ouvertures 115 prévues pour recevoir les alimentations du trocart, et/ou les câbles de maintien en position des caméras, comme les flexibles d'alimentation électrique des caméras.

De préférence encore, selon ce mode de réalisation, la portion tubulaire 113 de l'organe tubulaire 13 comprend un agencement avec glissières comme décrit plus haut, pour faciliter le déploiement des caméras de part et d'autre de l'endoscope. Le fonctionnement est identique au fonctionnement des modes de réalisation précédents. En effet, c'est l'insertion de l'endoscope 20 dans la portion tubulaire 10 du trocart qui va pousser les caméras (331;332) à l'extérieur de l'organe tubulaire 113. Les caméras (331;332) suivent une translation suivant les fentes 116 ménagées dans l'organe tubulaire 113 et servant de guide au déploiement.

Pour retirer le système d'imagerie du champ opératoire sans venir accidentellement heurter un organe, il suffit de retirer l'endoscope 20, puis de tirer sur les câbles pour successivement faire rentrer la première caméra 231 et la deuxième caméra 232 à l'intérieur de l'organe tubulaire 12, qui est lui-même retiré de l'incision.

Les caméras utilisées pour le deuxième dispositif d'imagerie 30 peuvent être variées tant qu'elles répondent aux problématiques de dimensions et de résolution requises. Par exemple, on peut utiliser des caméras CMOS miniatures (5 mm x 5 mm x 3.8 mm) telle que proposée par la société ST Microelectronics, qui présentent l'avantage d'avoir une haute résolution (1600 x 1200 pixels), un taux de trame élevé (environs 30 trames par seconde), un niveau bruit/signal faible, un bon contrôle d'exposition (+81dB), un large champ de vision (51°), ainsi qu'une grande profondeur de champ.

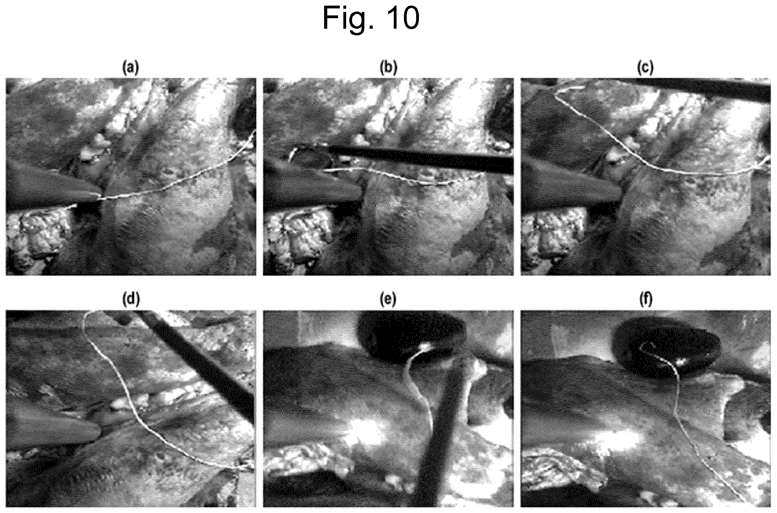

Une série d'essais a été effectuée pour tester l'efficacité du système d'imagerie multi-vision proposé par rapport à l'utilisation d'endoscopes traditionnels. A cette fin, il a été réalisé un banc d'essai très proche d'une table d'opération utilisant des organes de porc charcutier. Le scénario étudié a consisté à réaliser une succession de tâches souvent rencontrées dans le cas d'une chirurgie laparoscopique, notamment de la prostate. Dans un premier cas, le chirurgien a eu pour tâche de réaliser le scénario avec l'endoscope traditionnel (cf. figure 9) et ensuite reproduire les mêmes tâches avec le système d'imagerie intégrant la vision globale (cf. figure 10). Une dizaine de cycles ont été répétés pour évaluer qualitativement (qualité de la suture, confort pour le chirurgien/patient, etc.) et quantitativement (temps nécessaire, nombre d'ordres donnés à l'assistant(e)) les apports du système de vision globale par rapport à une utilisation d'un endoscope traditionnel.

Après une première analyse des résultats obtenus dans les deux cas de figures, c'est-à-dire, réaliser des points de suture à l'aide de l'endoscope traditionnel ou en utilisant le système de vision globale développé, il en est sorti qu'il fallait une moyenne de 3,8 minutes pour réussir la tâche en vision endoscopique et seulement 29 secondes dans le cas de l'utilisation de la vision globale. Ceci représente un gain de temps de l'ordre de 10. Pour une série de tests (5 tâches), 19 minutes sont nécessaires au praticien avec l'endoscope, tandis que 2,45 minutes suffisent avec le système de vision globale. Il en ressort que pour la procédure étudiée, il a été observé un gain de plus de 16 minutes avec le système intégrant la vision global par rapport à l'endoscope traditionnel, ce qui est très avantageux pour les interventions chirurgicales.

Outre le fait que le système d'imagerie proposé est très simple et facilite grandement le travail du chirurgien, le rendant ainsi plus efficace, il présente également l'avantage de pouvoir être utilisé avec tout type d'endoscope. En effet, la configuration du deuxième dispositif d'imagerie est indépendante de l'endoscope, puisqu'elle est très liée au trocart.

En outre si le deuxième dispositif d'imagerie pourrait par construction être conçu d'un seul tenant avec le trocart, il est également possible d'utiliser une configuration directement adaptable sur des trocarts existants, ou connectables par l'intermédiaire d'un adaptateur.

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée du système d'imagerie multi-vision présenté.

### REFERENCES BIBLIOGRAPHIQUES

- US 5,305,121
- US 6,614,595
- US 2012/0065468
- US 2011/0306832

## Revendications

1. Système d'imagerie multi-vision pour la chirurgie laparoscopique, comprenant :
- Un organe tubulaire (12), ledit organe tubulaire (12) ayant un axe longitudinal, une extrémité distale et une extrémité proximale ;
- Un premier dispositif d'imagerie (20) ayant un corps longitudinal (21) et une extrémité active (22) pour l'acquisition d'images, le premier dispositif d'imagerie (20) étant destiné à être inséré à travers l'organe tubulaire (12) avec l'extrémité active (22) en saillie par rapport à l'extrémité distale, et le premier dispositif d'imagerie (20) étant mobile dans l'organe tubulaire (12) suivant une translation selon l'axe longitudinal et/ou suivant une rotation autour de l'axe longitudinal ;
**caractérisé en ce qu'**il comprend en outre :
- Un deuxième dispositif d'imagerie (30) comprenant au moins deux caméras (31,131,231,331;32,132,232,332) montées chacune sur un organe de support (33,133,233,333), étant non couplées au premier dispositif d'imagerie (20), et étant déplaçables par rapport à l'organe tubulaire (12) entre :
∘ une position escamotée dans laquelle les caméras sont positionnées à l'intérieur de l'organe tubulaire, et
∘ une position déployée dans laquelle les caméras (31,131,231,331; 32,132,232,332) sont positionnées à l'extérieur de l'organe tubulaire (12) au niveau de l'extrémité distale, les caméras (31,131,231,331; 32,132,232,332) étant agencées de part et d'autre de l'axe longitudinal de l'organe tubulaire (12) et étant maintenues fixes par rapport à l'organe tubulaire (12) par des moyens de maintien, de manière à suivre tout mouvement de l'organe tubulaire (12).

2. Système selon la revendication 1, dans lequel les moyens de maintien sont au moins en partie formés par la paroi des organes de support (33,133,233,333) destinée à venir en appui sur le corps longitudinal (21) du premier dispositif d'imagerie (20), en position déployée.

3. Système selon l'une quelconque des revendications 1 ou 2, dans lequel les moyens de maintien comprennent des organes de rappel pour maintenir les caméras en appui contre l'organe tubulaire, en position déployée.

4. Système selon l'une quelconque des revendications 1 à 3, dans lequel, en position escamotée, les caméras (131,231,331;132,232,332) sont alignées à l'intérieur de l'organe tubulaire (12) selon l'axe longitudinal, ou positionnées à l'intérieur de l'organe tubulaire (12) en regard l'une de l'autre de part et d'autre de l'axe longitudinal.

5. Système selon l'une quelconque des revendications 1 à 4, dans lequel, en position déployée, les caméras (31,131,231,331;32,132,232,332) sont positionnées de sorte à avoir un axe optique parallèle à l'axe longitudinal.

6. Système selon l'une quelconque des revendications 1 à 5, dans lequel, en position déployée, les caméras (31,131,231,331;32,132,232,332) sont positionnées de sorte que les axes optiques des caméras forment un angle compris entre 6° et 15°.

7. Système selon l'une quelconque des revendications 1 à 6, dans lequel, en position déployée, les caméras (31,131,231,331;32,132,232,332) sont positionnées de sorte que les axes optiques des caméras sont séparés d'une distance comprise entre 10 millimètres et 30 millimètres.

8. Système selon l'une quelconque des revendications 1 à 7, dans lequel, en position déployée, les caméras (31,131,231,331;32,132,232,332) sont positionnées de sorte que les axes optiques des caméras et l'axe longitudinal sont dans un même plan.

9. Système selon l'une quelconque des revendications 1 à 8, dans lequel les organes de support (33,133,233,333) sont montés en rotation par rapport à l'organe tubulaire (12), les organes de support (33,133,233,333) comprenant un ergot d'actionnement permettant au corps longitudinal (21) de déplacer en rotation les organes de support en vue de leur déploiement lors de l'insertion du premier dispositif d'imagerie (20) dans l'organe tubulaire (12).

10. Système selon l'une quelconque des revendications 1 à 9, dans lequel les organes de support (33,133,233,333) sont montés en translation par rapport à l'organe tubulaire (12), les organes de support (33,133,233,333) comprenant un ergot d'actionnement permettant au corps longitudinal (21) de déplacer en translation les organes de support (33,133,233,333) en vue de leur déploiement lors de l'insertion du premier dispositif d'imagerie (20) dans l'organe tubulaire (12).

11. Système selon la revendication 10, dans lequel l'organe tubulaire comprend deux rails (116) et les organes de support (133,333) des caméras comprennent un ergot de guidage destiné à coopérer avec les rails pour une translation guidée des organes de support des caméras par rapport à l'organe tubulaire.

12. Système selon l'une quelconque des revendications 1 à 11, dans lequel l'organe tubulaire (12) est un trocart (10,11) destiné à être positionné à travers une incision pratiquée dans le corps d'un patient.

13. Système selon l'une quelconque des revendications 1 à 11, dans lequel l'organe tubulaire (12) est un adaptateur interne (13) destiné à être inséré à l'intérieur d'une portion tubulaire (10) d'un trocart, le diamètre interne de l'organe tubulaire étant sensiblement le même que le diamètre externe de la portion tubulaire du trocart.

14. Système selon l'une quelconque des revendications 1 à 11, dans lequel l'organe tubulaire (12) est un adaptateur externe (13) à l'intérieur duquel une portion tubulaire (10) d'un trocart est destinée à être insérée, le diamètre externe de l'organe tubulaire étant sensiblement le même que le diamètre interne de la portion tubulaire du trocart.

15. Système selon l'une quelconque des revendications 1 à 14, dans lequel le premier dispositif d'imagerie (20) est un endoscope.

## Patentansprüche

1. Multivisions-Bildgebungssystem für die laparoskopische Chirurgie, umfassend:
- ein rohrförmiges Organ (12), wobei das rohrförmige Organ (12) eine Längsachse, ein distales Ende und ein proximales Ende aufweist;
- eine erste Bildgebungsvorrichtung (20), die einen länglichen Körper (21) und ein aktives Ende (22) für die Bilderfassung aufweist, wobei die erste Bildgebungsvorrichtung (20) dazu vorgesehen ist, mit dem aktiven Ende (22) in Bezug auf das distale Ende vorspringend durch das rohrförmige Organ (12) hindurch eingeführt zu werden, und wobei die erste Bildgebungsvorrichtung (20) im rohrförmigen Organ (12) gemäß einer Translationsbewegung entlang der Längsachse und/oder gemäß einer Drehungbewegung um die Längsachse herum beweglich ist;
**dadurch gekennzeichnet, dass** es weiter umfasst:
- eine zweite Bildgebungsvorrichtung (30), die mindestens zwei Kameras (31, 131, 231, 331; 32, 132, 232, 332) umfasst, die jede an einem Trägerorgan (33, 133, 233, 333) montiert sind, die nicht mit der ersten Bildgebungsvorrichtung (20) gekoppelt sind und in Bezug auf das rohrförmige Organ (12) verschiebbar sind zwischen:
∘ einer eingefahrenen Stellung, in der die Kameras im Inneren des rohrförmigen Organs positioniert sind, und
∘ einer ausgefahrenen Stellung, in der die Kameras (31, 131, 231, 331; 32, 132, 232, 332) außerhalb des rohrförmigen Organs (12) im Bereich des distalen Endes positioniert sind, wobei die Kameras (31, 131, 231, 331; 32, 132, 232, 332) auf beiden Seiten der Längsachse des rohrförmigen Organs (12) angeordnet sind und in Bezug auf das rohrförmige Organ (12) von Haltemitteln gehalten werden, sodass sie jeder Bewegung des rohrförmigen Organs (12) folgen.

2. System nach Anspruch 1, wobei die Haltemittel mindestens zum Teil von der Wand der Trägerorgane (33, 133, 233, 333) gebildet werden, die dazu vorgesehen ist, in ausgefahrener Stellung am länglichen Körper (21) der ersten Bildgebungsvorrichtung (20) in Anlage zu gehen.

3. System nach einem der Ansprüche 1 oder 2, wobei die Haltemittel Rückstellorgane umfassen, um die Kameras in ausgefahrener Stellung gegen das rohrförmige Organ in Anlage zu halten.

4. System nach einem der Ansprüche 1 bis 3, wobei die Kameras (131, 231, 331; 132, 232, 332) in eingefahrener Stellung im Inneren des rohrförmigen Organs (12) entlang der Längsachse ausgerichtet sind, oder im Inneren des rohrförmigen Organs (12) auf beiden Seiten der Längsachse einander zugewandt positioniert sind.

5. System nach einem der Ansprüche 1 bis 4, wobei die Kameras (31, 131, 231, 331; 32, 132, 232, 332) in ausgefahrener Stellung so positioniert sind, dass sie eine zur Längsachse parallele optische Achse aufweisen.

6. System nach einem der Ansprüche 1 bis 5, wobei die Kameras (31, 131, 231, 331; 32, 132, 232, 332) in ausgefahrener Stellung so positioniert sind, dass die optischen Achsen der Kameras einen Winkel im Bereich zwischen 6° und 15° bilden.

7. System nach einem der Ansprüche 1 bis 6, wobei die Kameras (31, 131, 231, 331; 32, 132, 232, 332) in ausgefahrener Stellung so positioniert sind, dass die optischen Achsen der Kameras um einen Abstand im Bereich zwischen 10 Millimeter und 30 Millimeter getrennt sind.

8. System nach einem der Ansprüche 1 bis 7, wobei die Kameras (31, 131, 231, 331; 32, 132, 232, 332) in ausgefahrener Stellung so positioniert sind, dass die optischen Achsen der Kameras und die Längsachse in ein und derselben Ebene liegen.

9. System nach einem der Ansprüche 1 bis 8, wobei die Trägerorgane (33, 133, 233, 333) in Bezug auf das rohrförmige Organ (12) drehbeweglich montiert sind, wobei die Trägerorgane (33, 133, 233, 333) einen Betätigungsstift umfassen, der es dem länglichen Körper (21) ermöglicht, die Trägerorgane im Hinblick auf deren Ausfahren beim Einführen der ersten Bildgebungsvorrichtung (20) in das rohrförmige Organ (12) drehbeweglich zu verschieben.

10. System nach einem der Ansprüche 1 bis 9, wobei die Trägerorgane (33, 133, 233, 333) in Bezug auf das rohrförmige Organ (12) translationsbeweglich montiert sind, wobei die Trägerorgane (33, 133, 233, 333) einen Betätigungsstift umfassen, der es dem länglichen Körper (21) ermöglicht, die Trägerorgane (33, 133, 233, 333) im Hinblick auf deren Ausfahren beim Einführen der ersten Bildgebungsvorrichtung (20) in das rohrförmige Organ (12) translationsbeweglich zu verschieben.

11. System nach Anspruch 10, wobei das rohrförmige Organ zwei Schienen (116) umfasst, und die Trägerorgane (133, 333) der Kameras einen Führungsstift umfassen, der dazu vorgesehen ist, für eine geführte Translationsbewegung der Trägerorgane der Kameras in Bezug auf das rohrförmige Organ mit den Schienen zusammenzuwirken.

12. System nach einem der Ansprüche 1 bis 11, wobei das rohrförmige Organ (12) ein Trokar (10, 11) ist, der dazu vorgesehen ist, durch einen Einschritt hindurch positioniert zu werden, der im Körper eines Patienten vorgenommen ist.

13. System nach einem der Ansprüche 1 bis 11, wobei das rohrförmige Organ (12) ein Innenadapter (13) ist, der dazu vorgesehen ist, in das Innere eines rohrförmigen Abschnitts (10) eines Trokars eingeführt zu werden, wobei der Innendurchmesser des rohrförmigen Organs im Wesentlichen derselbe ist wie Außendurchmesser des rohrförmigen Abschnitts des Trokars.

14. System nach einem der Ansprüche 1 bis 11, wobei das rohrförmige Organ (12) ein Außenadapter (13) ist, in dessen Innerem ein rohrförmiger Abschnitt (10) eines Trokars dazu vorgesehen ist, eingeführt zu werden, wobei der Außendurchmesser des rohrförmigen Organs im Wesentlichen derselbe ist wie der Innendurchmesser des rohrförmigen Abschnitts des Trokars.

15. System nach einem der Ansprüche 1 bis 14, wobei die erste Bildgebungsvorrichtung (20) ein Endoskop ist.

## Claims

1. Multi-vision imaging system for laparoscopic surgery, comprising:
- A tubular member (12), said tubular member (12) having a longitudinal axis, a distal end and a proximal end;
- A first imaging device (20) having a longitudinal body (21) and an active end (22) for acquiring images, the first imaging device (20) being configured to be inserted through the tubular member (12), with the active end (22) protruding with respect to the distal end, and the first imaging device (20) being mobile in the tubular member (12) following a translation along the longitudinal axis and/or following a rotation around the longitudinal axis;
**characterised in that** it further comprises:
- A second imaging device (30) comprising at least two cameras (31, 131, 231, 331; 32, 132, 232, 332) each mounted on a support member (33, 133, 233, 333), being non-coupled to the first imaging device (20), and being moveable with respect to the tubular member (12) between:
∘ a retracted position wherein the cameras are positioned inside the tubular member, and
∘ a deployed position wherein the cameras (31, 131, 231, 331; 32, 132, 232, 332) are positioned outside of the tubular member (12) at the distal end, the cameras (31, 131, 231, 331; 32, 132, 232, 332) being arranged on either side of the longitudinal axis of the tubular member (12) and being held fixed with respect to the tubular member (12) by holding means, so as to follow any movement of the tubular member (12).

2. System according to claim 1, wherein the holding means are at least partially formed by the wall of the support members (33, 133, 233, 333) configured to bear against the longitudinal body (21) of the first imaging device (20), in a deployed position.

3. System according to any one of claims 1 or 2, wherein the holding means comprise return members to hold the cameras bearing against the tubular member, in a deployed position.

4. System according to any one of claims 1 to 3, wherein, in a retracted position, the cameras (131, 231, 331; 132, 232, 332) are aligned inside the tubular member (12) along the longitudinal axis, or positioned inside the tubular member (12) opposite each other, either side of the longitudinal axis.

5. System according to any one of claims 1 to 4, wherein, in a deployed position, the cameras (31, 131, 231, 331; 32, 132, 232, 332) are positioned so as to have an optical axis parallel to the longitudinal axis.

6. System according to any one of claims 1 to 5, wherein, in a deployed position, the cameras (31, 131, 231, 331; 32, 132, 232, 332) are positioned such that the optical axes of the cameras form an angle of between 6° and 15°.

7. System according to any one of claims 1 to 6, wherein, in a deployed position, the cameras (31, 131, 231, 331; 32, 132, 232, 332) are positioned such that the optical axes of the cameras are separated by a distance of between 10 millimetres and 30 millimetres.

8. System according to any one of claims 1 to 7, wherein, in a deployed position, the cameras (31, 131, 231, 331; 32, 132, 232, 332) are positioned such that the optical axes of the cameras and the longitudinal axis are in one same plane.

9. System according to any one of claims 1 to 8, wherein the support members (33, 133, 233, 333) are mounted in rotation with respect to the tubular member (12), the support members (33, 133, 233, 333) comprising an actuation lug make it possible for the longitudinal body (21) to move in rotation the support members with a view to them being deployed during the insertion of the first imaging device (20) in the tubular member (12).

10. System according to any one of claims 1 to 9, wherein the support members (33, 133, 233, 333) are mounted in translation with respect to the tubular member (12), the support members (33, 133, 233, 333) comprising an actuation lug making it possible for the longitudinal body (21) to move in translation the support members (33, 133, 233, 333) with a view to them being deployed during the insertion of the first imaging device (20) in the tubular member (12).

11. System according to claim 10, wherein the tubular member comprises two rails (116) and the support members (133, 333) of the cameras comprise a guide lug configured to cooperate with the rails for a guided translation of the support members of the cameras with respect to the tubular member.

12. System according to any one of claims 1 to 11, wherein the tubular member (12) is a trocar (10, 11) configured to be positioned through an incision made in the body of a patient.

13. System according to any one of claims 1 to 11, wherein the tubular member (12) is an internal adapter (13) configured to be inserted inside a tubular portion (10) of a trocar, the internal diameter of the tubular member being substantially the same as the external diameter of the tubular portion of the trocar.

14. System according to any one of claims 1 to 11, wherein the tubular member (12) is an external adapter (13) inside which a tubular portion (10) of a trocar is configured to be inserted, the external diameter of the tubular member being substantially the same as the internal diameter of the tubular portion of the trocar.

15. System according to any one of claims 1 to 14, wherein the first imaging device (20) is an endoscope.
